# FASCICULE DE BREVET EUROPEEN

(11) **EP 3 005 432 B1**
(45) Date de publication et mention de la délivrance du brevet: **11.09.2019**
(21) Numéro de dépôt: 14728482.2
(22) Date de dépôt: 23.05.2014
(51) Int. Cl.: H01L 41/22, H01L 41/113, H01L 41/04, A61B 5/024

(54) **PROCEDE DE FABRICATION D'UN CAPTEUR PIEZOELECTRIQUE SOUPLE**
VERFAHREN ZUR HERSTELLUNG EINES FLEXIBLEN PIEZOELEKTRISCHEN SENSORS
METHOD FOR PRODUCING A FLEXIBLE PIEZOELECTRIC SENSOR

(30) Priorité: 24.05.2013 FR 1354708
(43) Date de publication de la demande: 13.04.2016
(73) Titulaire: Chambre de Commerce et d'Industrie de Region Paris Ile de France, 75008 Paris (FR); Bodycap, 14000 Caen (FR)
(72) Inventeur: VALBIN, Laurie, F-77500 Chelles (FR); ROUSSEAU, Lionel, F-94170 Le Perreux Sur Marne (FR); VERJUS, Fabrice, F-14480 Creully (FR)
(74) Mandataire: IPAZ
(86) Numéro de dépôt international: PCT/EP2014/060617
(87) Numéro de publication internationale: WO 2014/187937

(56) Documents cités:
- EP-A2- 1 126 530
- WO-A2-2012/145278
- JP-A- 2006 204 534
- US-A1- 2007 152 537
- FUJITA T ET AL: "Flexible Sensor for Human Monitoring System by Using P(VDF/TrFE) Thin Film", FIFTH INTERNATIONAL CONFERENCE ON EMERGING TRENDS IN ENGINEERING AND TECHNOLOGY, 5-7 NOVEMBER 2012, HIMEJI, JAPAN, 2012, pages 75-79, XP032355108, DOI: 10.1109/ICETET.2012.22

## Description

### Domaine technique

La présente invention concerne un procédé de fabrication d'un capteur piézoélectrique. Elle concerne aussi un capteur fabriqué par ce procédé de fabrication, et un procédé d'utilisation d'un tel capteur.

Un tel procédé de fabrication permet à un utilisateur de fabriquer un capteur piézoélectrique « souple ».

Le domaine de l'invention est plus particulièrement celui des capteurs MEMS (Microsystèmes électromécaniques) piézoélectriques, notamment pour la détection de vibrations, pulsations, ou déformations.

### Etat de la technique antérieure

On connaît des procédés de fabrication de capteurs piézoélectriques sur substrat souple, tel que par exemple décrit dans l'article intitulé « Flexible piezoelectric pressure sensors using oriented aluminium nitride thin films prepared on polyethylene terephthalate films » de Akiyama et al., JOURNAL OF APPLIED PHYSICS vol.100, 114318 (2006).

Dans ce procédé de fabrication selon l'état de l'art, on fabrique une structure très basique : deux électrodes des deux côtés d'un film flexible en PET.

Des inconvénients de ce procédé selon l'état de l'art sont que :
- ce procédé selon l'état de l'art n'est pas compatible avec une technologie de salle blanche CMOS, et
- Ce procédé selon l'état de l'art ne permet pas de fabriquer des structures complexes (forme du capteur en poutre, rond, carré, etc...)

Des procédés semblables, comportant une étape de recouvrement d'un empilement électrode/piézoélectrique/électrode par une couche de polymère et une étape de rétraction du substrat sont connus des documents EP 1 126 530 A2 ou WO 2012/145278 A2, par exemple.

Le but de la présente invention est de résoudre au moins un de ces inconvénients.

### Exposé de l'invention

Cet objectif est atteint avec un procédé de fabrication d'un capteur piézoélectrique selon la revendication indépendante 1, comprenant les étapes suivantes :
- on fabrique, sur un support (comprenant de préférence une couche de silicium et/ou une couche d'oxyde de silicium ; ce support ou ces couches ayant de préférence une épaisseur totale d'au moins 400 micromètres), une superposition de couches de capteur, les couches de capteur comprenant une couche de matière piézoélectrique comprise entre une première électrode et une deuxième électrode, la première électrode n'étant pas en contact de la deuxième électrode, puis
- alors que les couches de capteur sont toujours portées par le support, on recouvre les couches de capteur par une première couche de polymère, puis
- on retire, de la superposition de couches de capteur, au moins une partie (de préférence la totalité) du support, de préférence de sorte que les couches de capteur recouvertes par la première couche de polymère ne soient plus portées par le support.

Le retrait d'au moins une partie (de préférence de la totalité) du support comprend une destruction d'au moins une partie (de préférence de la totalité) du support. La suppression de la totalité du support permet d'obtenir un capteur piézoélectrique souple et particulièrement fin. Ce capteur est, en outre, autosupporté.

On peut fabriquer, après le retrait de l'au moins une partie du support, une deuxième couche de polymère de sorte que les couches de capteur soient encapsulées entre la première couche de polymère et la deuxième couche de polymère.

L'encapsulation des couches de capteur par la première couche de polymère et la deuxième couche de polymère permet d'une part de protéger le capteur des agressions extérieures tout en conservant sa souplesse et d'autre part de rendre le capteur biocompatible et autosupporté.

Le polymère utilisé pour la première couche de polymère et/ou la deuxième couche de polymère selon l'invention peut être sélectionné parmi la liste constituée de Parylène ou de Polyimide.

De préférence, la première couche de polymère et/ou la deuxième couche de polymère est constituée de Parylène car il évite la présence de zone de fragilité et son dépôt n'altère pas le capteur selon l'invention. De préférence, la première couche de polymère et la deuxième couche de polymère sont constituées du même polymère. L'épaisseur de la première couche de polymère est d'au moins 3 µm au-dessus du capteur. L'épaisseur de la deuxième couche de polymère est d'au moins 3 µm en dessous du capteur. L'épaisseur de chacune des couches de polymère est généralement inférieure à 50 µm et de préférence inférieure à 20 µm, de manière plus préférentielle inférieure à 10 µm.

Le support est de préférence un support rigide. A des conditions standards ambiantes de température de 25 °C et de pression absolue de 0.986 atm = 1 bar (unité de pression atmosphère normale), le support rigide a de préférence un module d'Young d'au moins 50 GPa (gigapascal), de préférence d'au moins 100GPa, de préférence compris entre 160 GPa et 180 GPa. Comme cela est explicité dans les exemples, le support comprend de préférence du silicium comme par exemple du verre et de façon plus préférée il est constitué de silicium. Dans ce cas, le monocristal de silicium peut présenter une surface oxydée. Par rapport à un support en métal, ce type de support permet une mise en oeuvre de l'invention en salle blanche (utilisant la technologie CMOS) et également d'obtenir un capteur présentant des structures plus variées et, par rapport à un support en polymère, d'obtenir un capteur plus fin et une mise en oeuvre plus aisée.

Selon la présente invention, la superposition de couches de capteur comprend une première couche conductrice et une deuxième couche conductrice, chacune des première et deuxième couches conductrices comprenant deux parties, une première partie de la première couche conductrice étant en contact avec une première partie de la deuxième couche conductrice, une deuxième partie de la première couche conductrice étant en contact avec une deuxième partie de la deuxième couche conductrice, la première électrode étant formé par la première partie de la première couche conductrice et par la première partie de la deuxième couche conductrice, la deuxième électrode étant formé par la deuxième partie de la première couche conductrice et par la deuxième partie de la deuxième couche conductrice, la couche de matière piézoélectrique étant comprise au moins en partie entre la première couche conductrice et la deuxième couche conductrice. Ce mode de réalisation avantageux permet notamment une plus grande liberté dans la géométrie accordée au capteur et une utilisation possible de l'aluminium pour chaque couche conductrice.

Les couches conductrices sont de préférence métalliques. Les couches conductrices peuvent être par exemple en platine, or ou aluminium. L'utilisation d'aluminium permet de réduire les coûts de fabrication des capteurs tandis que le platine est plus adapté aux applications *in vivo.*

Le procédé selon l'invention peut comprendre en outre une fabrication d'une antenne reliant la première électrode à la deuxième électrode, ladite antenne faisant un ou plusieurs tours de manière à dessiner une boucle fermée. De façon préférée, l'épaisseur de l'antenne est comprise entre 10 et 50 µm. L'antenne permet avantageusement d'éviter la présence de trou à travers la première couche de polymère (i.e. pour l'accès aux électrodes) et ainsi d'encapsuler complétement le capteur.

Le procédé selon l'invention peut être mené à une température inférieure à 250 °C et ainsi permettre la fabrication du capteur sur un support intégrant des circuits intégrés. Le support peut, dans certains cas, avoir préalablement subi des étapes de préparation incluant des passages à des températures supérieures à 250°C.

La couche piézoélectrique peut avoir une forme de poutre s'étendant longitudinalement, et la poutre peut s'étendre longitudinalement à partir du périmètre de la boucle et vers l'intérieur de la boucle. Ce mode de réalisation permet notamment de mesurer des déformations sur de plus grandes surfaces et par exemple de réaliser des mesures de déformation longitudinales, transversales ainsi que de rayons de courbure.

Comme cela a été précisé, l'invention porte notamment sur un procédé compatible avec une technologie de salle blanche CMOS. Ainsi la couche piézoélectrique est de préférence constituée de PbZrTiO₃ (PZT, Titano-Zirconate de Plomb), nitrure d'aluminium (AIN) ou de PVDF (Polyfluorure de vinylidène). De façon plus préférée, comme cela est présenté dans les exemples, la couche piézoélectrique est constituée d'AIN. Une couche AIN, contrairement à une couche en PbZrTiO₃, est compatible avec la technologie CMOS et elle n'est pas toxique.

Les couches de capteur peuvent comprendre :
- une couche isolante, réalisée entre l'antenne et la première électrode et réalisée entre l'antenne et la deuxième électrode, au niveau de zones où l'antenne est superposée à la première électrode ou à la deuxième électrode. La couche isolante peut être réalisée dans une matière isolante électriquement, ayant une résistivité électrique supérieure ou égale à 10³ ohm.cm (de préférence à 10⁶ ohm.cm) à des conditions standards ambiantes de température de 25 °C et de pression absolue de 0.986 atm ; et/ou
- une couche de rigidification superposée à la couche piézoélectrique.

La couche de rigidification a, aux conditions standards de température et de pression ambiante de 25°C et de pression absolue de 0.986 atm., un module d'Young d'au moins 50GPa, de préférence d'au moins 100GPa, de préférence compris entre 100 GPa et 1 TPa.

La couche de rigidification et la couche isolante sont de préférence réalisées dans une même matière au cours d'une même étape de dépôt de couche.

L'épaisseur totale des couches de capteur est de préférence inférieure à 3 micromètres.

Comme cela est présenté dans les exemples, lorsqu'il est encapsulé dans un polymère, l'épaisseur finale du capteur comprenant une antenne est comprise entre 10 et 50 µm. En l'absence d'antenne, l'épaisseur du capteur encapsulé peut être inférieure à 50 µm, de préférence inférieure à 10 µm. En l'absence d'antenne, l'épaisseur du capteur encapsulé est par exemple de 10 µm.

Suivant encore un autre aspect de l'invention, il est proposé un capteur obtenu par un procédé de fabrication selon l'invention.

En particulier, suivant encore un autre aspect de l'invention, il est proposé un capteur piézoélectrique comprenant une couche de matière piézoélectrique comprise entre une première électrode et une deuxième électrode, la première électrode n'étant pas en contact de la deuxième électrode, caractérisé en ce que le dit capteur est entre deux couches de polymère et qu'il présente une épaisseur, incluant les couches de polymère, inférieure à 50 µm, de préférence inférieure à 10 µm.

De façon préférée, ledit capteur comprend une couche de matière piézoélectrique constituée de matériau AIN.

Suivant encore un autre aspect de l'invention, il est proposé un procédé d'utilisation d'un capteur obtenu selon le procédé de fabrication selon l'invention, caractérisé en ce que l'on applique le capteur sur ou dans une structure afin d'en mesurer au moins une contrainte ou au moins une pression appliquée sur cette structure.

Suivant encore un autre aspect de l'invention, il est proposé un procédé d'utilisation d'un capteur obtenu selon le procédé de fabrication selon l'invention, caractérisé en ce qu'il comprend les étapes suivantes :
- on applique le capteur sur un corps humain ou animal, puis
- pour chaque pulsation cardiaque dudit corps parmi plusieurs pulsations, la pulsation cardiaque déforme la couche piézoélectrique, engendrant une variation de charge électrique dans les électrodes.
- pour chaque variation de charge électrique, on détecte cette variation de charge électrique, et
- on déduit, à partir de ces détections, une fréquence cardiaque du corps.

Suivant encore un autre aspect de l'invention, il est proposé un procédé d'utilisation d'un capteur obtenu selon le procédé de fabrication selon l'invention, caractérisé en ce qu'il comprend les étapes suivantes :
- on applique le capteur sur un corps humain ou animal, puis
- on émet un champ électromagnétique par une électronique d'excitation, puis
- le champ électromagnétique est capté par une antenne connectée électriquement à la première électrode et à la deuxième électrode du capteur, engendrant dans l'antenne un courant électrique à une fréquence f1 égale à une fréquence f0 de résonance du capteur au repos ou à une harmonique de cette fréquence f0 de résonnance,
- pour chaque pulsation cardiaque dudit corps parmi plusieurs pulsations, la pulsation cardiaque déforme la couche piézoélectrique, provoquant une variation Df de la fréquence de résonance,
- pour chaque variation Df de la fréquence de résonance, on réceptionne par une électronique de mesure un signal oscillant à une fréquence f1 + Df,
- on déduit, à partir de ces détections, une fréquence cardiaque du corps.

### Description des figures et modes de réalisation

D'autres avantages et particularités de l'invention apparaîtront à la lecture de la description détaillée de mises en oeuvre et de modes de réalisation nullement limitatifs, et des dessins annexés suivants :

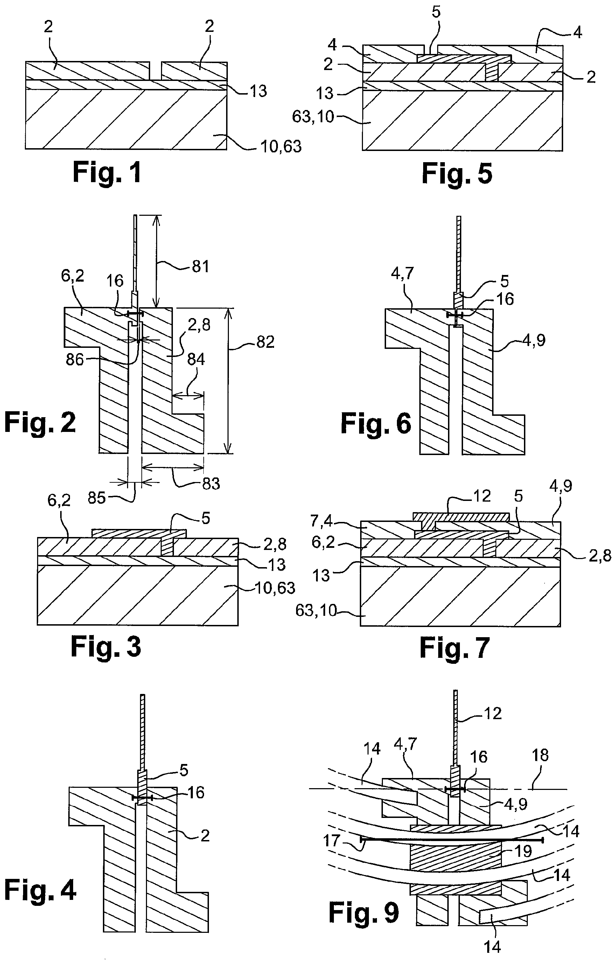

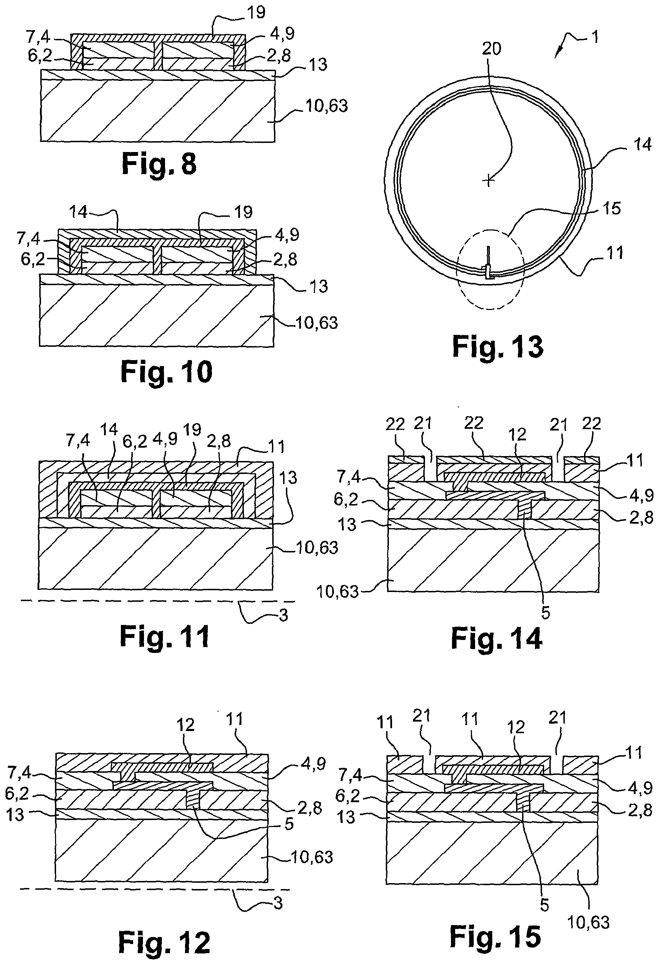

- les figures 2, 4, 6, 9, 13 sont des vues de dessus, parallèlement au plan de couche référencé 3 sur la figure 11, de différentes couches à différentes étapes d'un premier mode de réalisation de procédé selon l'invention de fabrication d'un capteur 1,
- les figures 1, 3, 5, 7 et 12 sont des vues de coupe de profil (perpendiculairement au plan de couche 3) de différentes couches à différentes étapes du premier mode de réalisation de procédé de fabrication selon l'invention ; ces vues de coupe illustrent les différentes couches le long du premier segment de coupe 16 illustré sur les figures 2, 4, 6, 9,
- les figures 8, 10, 11 sont des vues de coupe de profil (perpendiculairement au plan de couche 3) de différentes couches à différentes étapes du premier mode de réalisation de procédé de fabrication selon l'invention ; ces vues de coupe illustrent les différentes couches le long du deuxième segment de coupe 17 illustré sur la figure 9,
- les figures 14 et 15 sont des vues de coupe de profil (perpendiculairement au plan de couche 3) de différentes couches à différentes étapes d'une variante (par rapport à la variante avec antenne 14) du premier mode de réalisation de procédé de fabrication selon l'invention ; ces vues de coupe illustrent les différentes couches le long du premier segment de coupe 16 illustré sur les figures 2, 4, 6, 9,

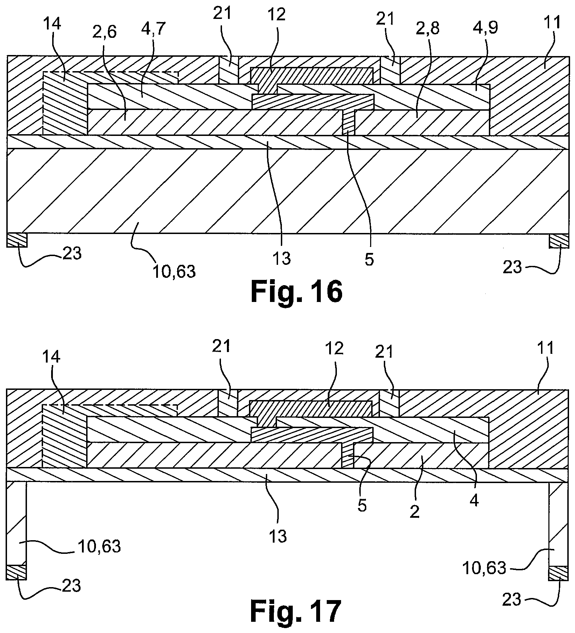

- les figures 16 à 19 sont des vues de coupe de profil (perpendiculairement au plan de couche 3) de différentes couches à différentes étapes du premier mode de réalisation de procédé de fabrication selon l'invention ; ces vues de coupe illustrent les différentes couches le long de l'axe de coupe 18 illustré sur la figure 9,

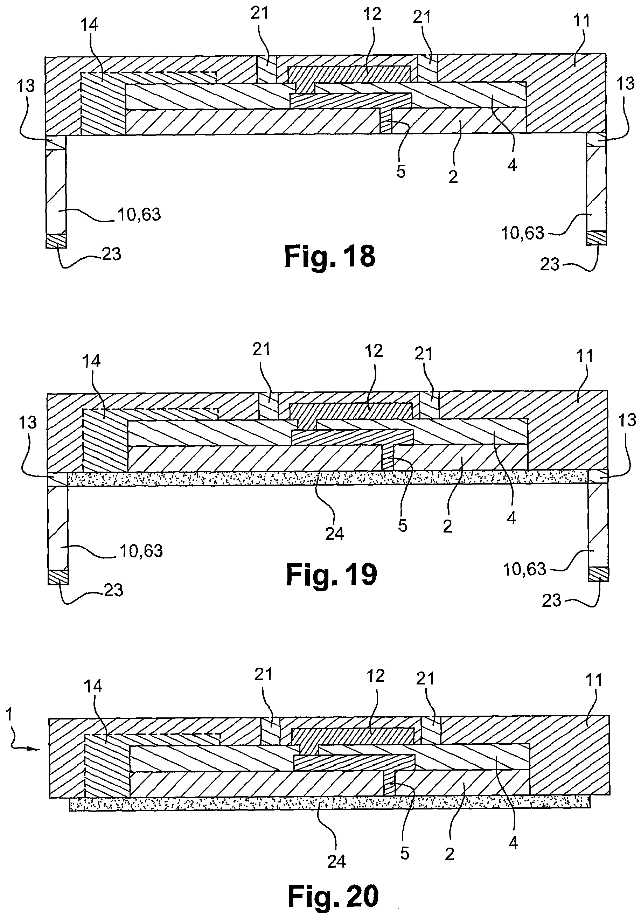

- la figure 20 est une vue de coupe de profil (perpendiculairement au plan de couche 3) du capteur 1 obtenu par le premier mode de réalisation de procédé de fabrication selon l'invention ; cette vue de coupe illustre les différentes couches de ce capteur le long de l'axe de coupe 18 illustré sur la figure 9,

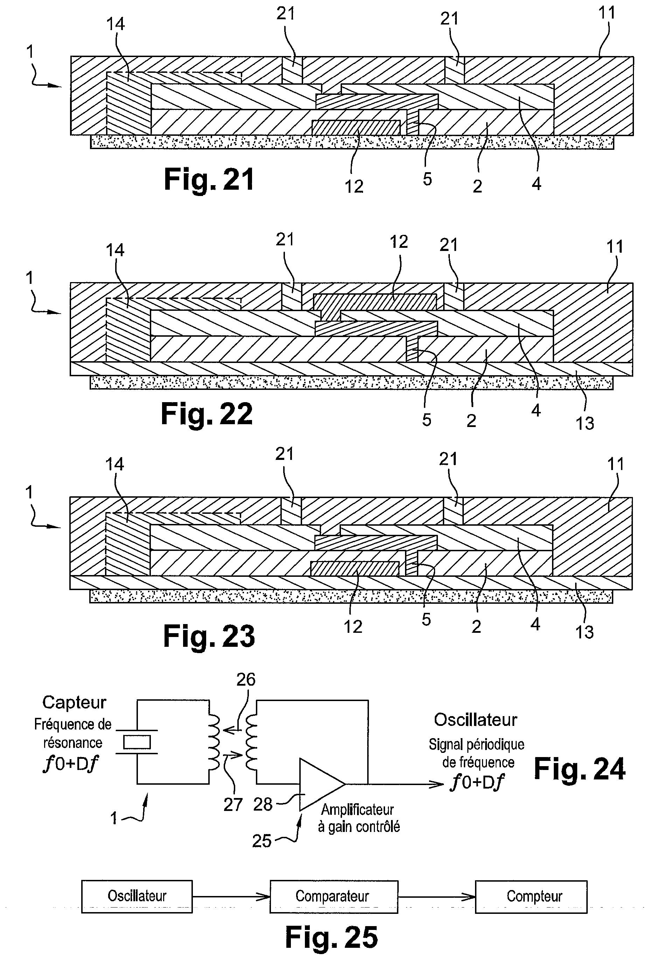

- les figures 21 à 23 sont des vues de coupe de profil (perpendiculairement au plan de couche 3) de différentes variantes du capteur 1 obtenu par différentes variantes du premier mode de réalisation de procédé de fabrication selon l'invention ; ces vues de coupe illustrent les différentes couches du capteur 1 le long de l'axe de coupe 18 illustré sur la figure 9,
- la figure 24 est un schéma d'une électronique pour l'utilisation du capteur 1,
- la figure 25 est un organigramme du principe de l'électronique pour l'utilisation du capteur 1,

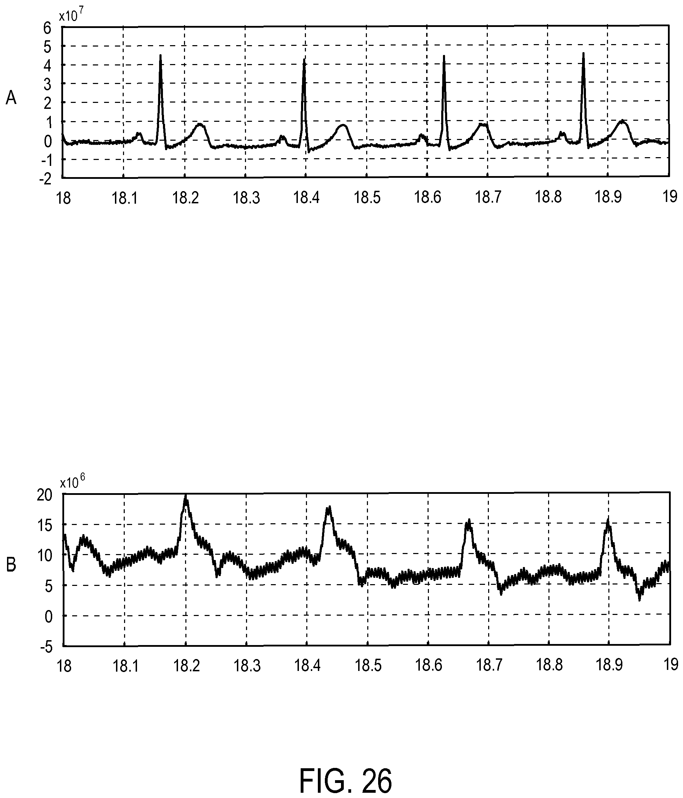

- La figure 26 est un exemple de l'utilisation du capteur 1 selon l'invention.

Dans la description ci-dessous, chaque épaisseur est définie perpendiculairement au plan de couche 3.

Les proportions respectives des épaisseurs et des largeurs des différentes couches ne sont pas respectées sur les vues de profil.

Ces modes de réalisation étant nullement limitatifs, on pourra notamment considérer des variantes de l'invention ne comprenant qu'une sélection de caractéristiques décrites par la suite isolées des autres caractéristiques décrites (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection comprend au moins une caractéristique de préférence fonctionnelle sans détails structurels, ou avec seulement une partie des détails structurels si cette partie uniquement est suffisante pour conférer un avantage technique ou à différencier l'invention par rapport à l'état de la technique antérieure.

On va tout d'abord décrire, en référence aux figures 1 à 20, un premier mode de réalisation de procédé de fabrication selon l'invention.

Ce premier mode de procédé selon l'invention pour fabriquer un capteur piézoélectrique 1 comprend les étapes suivantes :
- on fabrique, sur un support rigide 10, une superposition (selon une direction de superposition perpendiculaire à un plan de couche 3) de couches de capteur 2, 4, 5, 12, 19, chaque couche de capteur étant fabriquée par photolithographie, les couches de capteur comprenant une couche de matière piézoélectrique 5 (s'étendant au moins en partie parallèlement au plan de couche 3) comprise entre une première électrode 6, 7 (s'étendant au moins en partie parallèlement au plan de couche 3) et une deuxième électrode 8, 9 (s'étendant au moins en partie parallèlement au plan de couche 3), la première électrode n'étant pas en contact de la deuxième électrode, puis
- alors que les couches de capteur 2, 4, 5, 12, 19 sont toujours portées par le support rigide 10, on recouvre les couches de capteur par une couche de polymère 11, puis
- on retire du support rigide 10 la superposition de couches de capteur, de sorte que les couches de capteur recouvertes par la couche de polymère 11 ne soient plus portées par le support rigide 10.

Comme on le verra par la suite, ce procédé selon l'invention est compatible avec une technologie de salle blanche CMOS, et permet de fabriquer des structures complexes.

### Fabrication, sur le support rigide 10 de la superposition de couches de capteur 2, 4, 5, 12, 19

Pour la fabrication des couches de capteur 2, 4, 5, 12, 19, on utilise comme support rigide 10 une matière (de préférence du silicium) ayant, dans des conditions standards ambiantes de température de 25 °C et de pression absolue de 0.986 atm., un module d'Young d'au moins 50 GPa (gigapascal), plus particulièrement d'au moins 100GPa, de préférence compris entre 160 GPa et 180 GPa.

Dans cette description, toutes les valeurs de modules d'Young sont définies selon un axe perpendiculaire au plan de couche 3 illustré sur la figure 11.

Le support rigide 10 typiquement comprend (voir même de préférence consiste en) une couche 63 de silicium non oxydé.

Une couche 13 de silicium oxydé est en contact avec la couche 63 de silicium non oxydé. Selon la variante considérée, cette couche oxydée 13 fait partie du support rigide 10 ou fait partie des couches de capteur, selon que cette couche 13 soit conservée ou pas pour le capteur 1.

Le support rigide 10 possède, selon la direction de superposition perpendiculaire au plan de couche 3, une épaisseur d'au moins 400 µm.

La superposition des couches de capteur comprend une première couche 2 conductrice d'électricité (de préférence métallique, typiquement en aluminium) s'étendant parallèlement au plan de couche 3, et une deuxième couche 4 conductrice d'électricité (de préférence métallique, typiquement en aluminium) s'étendant parallèlement au plan de couche 3.

Chacune des première 2 et deuxième 4 couches conductrices a une résistivité électrique inférieure ou égale à 3 ohm.cm (de préférence à 10⁻⁴ ohm.cm voir même 10⁻⁶ ohm.cm).

Chacune des première 2 et deuxième 4 couches conductrices comprend deux parties : une première partie 6 de la première couche conductrice est en contact avec une première partie 7 de la deuxième couche conductrice ; une deuxième partie 8 de la première couche conductrice est en contact avec une deuxième partie 9 de la deuxième couche conductrice.

La première électrode 6, 7 est formée par la première partie 6 de la première couche conductrice et par la première partie 7 de la deuxième couche conductrice.

La deuxième électrode 8, 9 est formée par la deuxième partie 8 de la première couche conductrice et par la deuxième partie 9 de la deuxième couche conductrice.

La couche de matière piézoélectrique 5 est comprise au moins en partie entre la première couche conductrice 2 et la deuxième couche conductrice 4.

La couche de matière piézoélectrique 5 est en contact avec la première électrode (de préférence avec chacune des parties 6,7).

La couche de matière piézoélectrique 5 est en contact avec la deuxième électrode (de préférence avec chacune des parties 8,9).

La première électrode 6, 7 n'est pas en contact de la deuxième électrode 8, 9. Du courant électrique passant entre les deux électrodes doit nécessairement passer, parmi les couches de capteur, par l'intermédiaire de la couche 5 de matière piézoélectrique ou, en dehors des couches de capteur, par l'antenne 14 décrite par la suite.

En référence aux figures 1 et 2, la première couche conductrice 2 en Aluminium est fabriquée par photolithographie selon les étapes suivantes :
- Nettoyage d'une plaquette de silicium
- Oxydation de la plaquette de silicium à 1050°C avec un mélange de gaz H et O₂ (« oxydation humide ») / Epaisseur obtenue de la couche oxydée 13 égale à 1,6µm, sur la couche 63 de silicium non oxydé.
- Dépôt de métal (aluminium), épaisseur de 200 nm au-dessus de la couche 13, sous Argon : 0,5.10⁻² mbar (pression dans la chambre)/ pendant 5 min/ à 200 watt de puissance du générateur DC alimentant la cible d'aluminium
- Photolithographie de résine photosensible positive (PFR7790 de chez JSR) ; épaisseur 1.1 µm
   Vitesse d'étalement : 4500 tr/min pendant 30 secondes
   Recuit « doux » sur plaque chauffante (« Soft Bake ») : 3min à 110°C
   Exposition à travers un masque pendant 4 secondes à 6,5 mW/cm² de puissance lumineuse UV
   Développement de la résine (développeur PRD238 de chez OMG) pendant 1min
   Rinçage à l'Eau Déionisée
   Recuit sur plaque chauffante (« Hard Bake ») : 3min à 110°C
- Gravure liquide (Al etch ANT 760-30-150 de chez OMG) pour Attaque Aluminium
   Pendant 3 min / Rinçage à l'Eau Déionisée
- Enlèvement de la Résine : utilisation, comme dissolvant, d'Acétone puis isopropanol alcool puis eau déionisée.

Les dimensions référencées 81 à 86 sur la figure 2 ont les valeurs suivantes :
Référence 81 (raccourcie sur les vues de dessus des figures 2, 4, 6, 9 par rapport aux autres dimensions): 1 centimètre
Référence 82 : 2 millimètres
Référence 83 : 1 millimètre
Référence 84 : 0,5 millimètre
Référence 85 :0,2 millimètre
Référence 86 : 20 micromètres

En référence aux figures 3 et 4, la couche 5 de matière piézoélectrique en Nitrure d'aluminium est fabriquée par photolithographie sur la première couche conductrice 2 selon les étapes suivantes :
- Dépôt d'AIN (Nitrure d'Aluminium) par pulvérisation réactive
   Epaisseur de 1 µm au-dessus de la couche 2, sous :
   Argon : 1,9.10-2 mbar (pression partielle) et
   Azote : 3,4.10-2 mbar (pression partielle)
   pendant 1h / à 500W (puissance DC du générateur) / à 220°C (température du substrat) avec un vide résiduel avant dépôt inférieur à 33.3 mPa (2,5.10-7 Torr).
- Photolithographie de résine photosensible PFR7790
   épaisseur 1.1 µm
   Vitesse d'étalement : 4500 tr/min pendant 30 secondes
   Recuit « doux » sur plaque chauffante (« Soft Bake ») : 3min à 110°C
   Exposition à travers un masque pendant 4 secondes à 6,5 mW/cm² de puissance lumineuse UV
   Développement de la résine (développeur PRD238 de chez OMG) pendant 1min
   Rinçage à l'Eau Déionisée
   Recuit sur plaque chauffante (« Hard Bake ») : 3min à 110°C
- Gravure liquide (PRD 238 de chez OMG)
   Pendant 3 min / Rinçage à l'Eau Déionisée
- Enlèvement de la Résine : utilisation, comme dissolvant, d'Acétone puis isopropanol alcool puis eau déionisée.

De manière générale, on peut utiliser d'autres matériaux piézoélectriques, tels que PZT (Titano-Zirconate de Plomb), ou PVDF (Polyfluorure de vinylidène).

En référence aux figures 5 et 6, la deuxième couche conductrice 4 en Aluminium est fabriquée par photolithographie sur la première couche conductrice 2 et sur la couche 5 de matière piézoélectrique selon les étapes suivantes :
- Dépôt de métal (aluminium), épaisseur de 200 nm au-dessus de la couche 2 et de 200 nm au-dessus de la couche 5 (contrairement à la représentation schématique des vues de profil des figures 5, 7, 12, 14, 15 et 16 à 23, la face supérieure de la couche conductrice 4 sur ces figures n'est pas plane mais a une forme de marche de manière à avoir sensiblement une même épaisseur au-dessus de chacune des couches 2 et 5) sous Argon : 0,5.10⁻² mbar (pression dans la chambre)/ pendant 5 min/ à 200 watt de puissance du générateur DC alimentant la cible d'aluminium
- Photolithographie de résine photosensible positive (PFR7790 de chez JSR) ; épaisseur 1.1 µm
   Vitesse d'étalement : 4500 tr/min pendant 30 sec
   Recuit « doux » sur plaque chauffante (« Soft Bake ») : 3 minutes à 110°C
   Exposition à travers un masque pendant 4 secondes à 6,5 mW/cm² de puissance lumineuse UV
   Développement de la résine (développeur PRD238 de chez OMG) pendant 1min
   Rinçage à l'Eau Déionisée
   Recuit sur plaque chauffante (« Hard Bake ») : 3min à 110°
- Gravure liquide (Al etch ANT 760-30-150 de chez OMG) pour Attaque Aluminium
   Pendant 3 min / Rinçage à l'Eau Déionisée
- Enlèvement de la Résine : utilisation, comme dissolvant, d'Acétone puis isopropanol alcool puis eau déionisée.

En référence aux figures 7, 8 et 9, une couche de rigidification 12 en Nitrure (Si₃N₄) superposée à la couche piézoélectrique 5 est fabriquée par photolithographie en même temps qu'une couche isolante 19 en Nitrure (Si₃N₄). La couche isolante 19 est réalisée dans une matière isolante électriquement (ici en Nitrure (Si₃N₄)), ayant une résistivité électrique supérieure ou égale à 10³ ohm.cm (de préférence à 10⁶ ohm.cm) à des conditions standards ambiantes de température de 25 °C et de pression absolue de 0.986 atm. La couche de rigidification 12 en Nitrure (Si₃N₄) est fabriquée sur la deuxième couche conductrice 4 et sur la couche 5 de matière piézoélectrique (de préférence sur l'intégralité de la surface supérieure de la couche piézoélectrique) et la couche isolante 19 est fabriquée sur la deuxième couche conductrice 4 selon les étapes suivantes :
- Dépôt Nitrure (Si₃N₄) par dépôt en phase plasma PEVCD (« plasma enhanced Chemical vapor deposition »), épaisseur de cette couche : 1 µm au-dessus de la couche 4 et de la couche 19.
- Photolithographie de résine photosensible positive (PFR7790 de chez JSR) ; épaisseur 1.1 µm
   Vitesse d'étalement : 4500 tr/min pendant 30 secondes Recuit « doux » sur plaque chauffante (« Soft Bake ») : 3 minutes à 110°C
   Exposition à travers un masque pendant 4 secondes à 6,5 mW/cm² de puissance lumineuse UV
   Développement de la résine (développeur PRD238 de chez OMG) pendant 1min
   Rinçage à l'Eau Déionisée
   Recuit sur plaque chauffante (« Hard Bake ») : 3min à 110°C
- Gravure plasma RIE (gravure par ions réactifs ou « Reactive Ion Etching ») SF6 (Hexafluorure de soufre) 10 sccm de débit / 2.67 Pa (20 mTorr) de pression partielle dans la chambre / 30 Watt de puissance du générateur radio fréquence
- Enlèvement de la Résine : utilisation, comme dissolvant, d'Acétone puis isopropanol alcool puis eau déionisée.

Ainsi la couche de rigidification 12 et la couche isolante 19 sont réalisées dans une même matière au cours d'un même étape de dépôt de couche par photolithographie.

La couche de rigidification 12 a, aux conditions standards de température et de pression ambiante de 25 °C et de pression absolue de 0.986 atm., un module d'Young supérieur à celui du support rigide 10.

La couche de rigidification 12 a, aux conditions standards de température et de pression ambiante de 25 °C et de pression absolue de 0.986 atm., un module d'Young d'au moins 50GPa, plus particulièrement d'au moins 100GPa, de préférence compris entre 100 GPa et 1 TPa (Térapascal). Le rôle de cette couche de rigidification 12 est de définir les propriétés mécaniques de vibrations de la couche piézoélectrique, notamment sa fréquence de résonance.

La couche de rigidification à une épaisseur d'au moins 1 µm au-dessus de la couche 4 et de la couche 19.

En référence aux figures 9, 10 et 13 (la figure 9 correspondant à l'agrandissement de la zone 15 délimitée sur la figure 13), le premier mode de réalisation de procédé selon l'invention comprend en outre une fabrication d'une antenne 14 reliant la première électrode 6, 7 à la deuxième électrode 8, 9, ladite antenne 14 faisant au moins un (de préférence plusieurs) tours autour d'un centre 20 (par exemple trois tours comme illustré sur la figure 13) de manière à dessiner une boucle fermée.

L'antenne 14 métallique (typiquement en cuivre ou en or) est fabriquée par électrodéposition sur la première électrode 7 et sur la deuxième électrode 9 (plus exactement sur la deuxième couche conductrice 4) et sur la couche isolante 19, selon les étapes suivantes :
- Réalisation d'une couche d'accroche (« Seed layer ») fine (épaisseur comprise entre 50 et 100 nm) typiquement en Cuivre ou en or
- définition du moule en résine épaisse
- dépôt électrolytique de cuivre ou or
- Enlèvement du moule de résine

L'épaisseur de l'antenne 14 est comprise entre 10 et 50 µm au-dessus de la couche 13.

Dans le cas illustré sur la figure 13 ou la couche de matière piézoélectrique 5 a une forme de poutre s'étendant longitudinalement, cette poutre s'étend longitudinalement parallèlement au plan de couche 3 à partir du périmètre de la boucle et vers l'intérieur de la boucle, de préférence vers le centre 20 de la boucle.

L'antenne comprend deux extrémités et fait N tours autour du centre 20 (avec N un entier naturel) : une première de ses extrémités est connectée électriquement à la première électrode 6, 7, une deuxième de ses extrémités est connectée à la deuxième électrode 8,9, et l'antenne 14 passe N-1 fois au-dessus de la première électrode et de la deuxième électrode sans être en contact de ces électrodes pour chacun de ces passages. En effet, la couche isolante 19 est réalisée entre l'antenne 14 et la première électrode 6, 7 et réalisée entre l'antenne 14 et la deuxième électrode 8, 9, au niveau de chacun de ces N-1 passages où l'antenne 14 est superposée à la première électrode 6, 7 ou à la deuxième électrode 8, 9 de manière à éviter tout cours circuit entre la première électrode 6,7 et la deuxième électrode 8,9.

L'épaisseur totale des couches de capteur 2, 4, 5, 12, 19 (et donc sans l'antenne 14 et sans la couche de polymère 11) est inférieure à 3 micromètres.

### Recouvrement des couches de capteur par la couche de polymère 11

Pour la couche 11 (de préférence ainsi que pour la couche 24 introduite par la suite) on utilise un polymère qui, aux conditions standards de température et de pression ambiante de 25 °C et de pression absolue de 0.986 atm., possède un module d'Young inférieur à 50 GPa, plus particulièrement inférieur à 1MPa (mégapascal), de préférence compris entre 0.3 MPa et 1MPa.

L'épaisseur de la couche 11 de polymère est au minimum de 3 µm au-dessus des couches de capteur 2, 4, 5, 12, 19.

La couche de polymère 11 est fabriquée par un dépôt de Parylène (équipement SCS) ou de Polyimide 2611 (Epaisseur finale entre 20 et 60µm au-dessus de la couche 13, selon l'épaisseur de l'antenne 14 comprise entre 10 et 50 µm au-dessus de la couche 13).

Les première 6, 7 et deuxième 8, 9 électrodes et la couche piézoélectrique 5 (de même que l'antenne 14 la couche de rigidification 12 et la couche isolante 19) sont situées entre la couche de polymère 11 et le support rigide 10.

### Variante du premier mode de réalisation de procédé selon l'invention

En référence aux figures 14 et 15, dans une variante du premier mode de réalisation de procédé de fabrication selon l'invention dont les premières étapes viennent d'être décrites, l'antenne 14 et la couche isolante 19 ne sont pas fabriquées.

A la place, après la fabrication de la couche de polymère 11, on « libère » une partie de la première électrode 6, 7 en enlevant une partie de la couche de polymère 11 en contact avec la première électrode 6, 7 et on « libère » une partie de la deuxième électrode 8, 9 en enlevant une partie de la couche de polymère 11 en contact avec la deuxième électrode 8, 9.

Pour cela, on réalise pour chacune des électrodes un trou 21 à travers la couche de polymère 11 permettant l'accès à chacune de ces électrodes pour une connexion électrique depuis l'extérieur du capteur 1.

Chacun de ces trous 21 est réalisé (de préférence de manière simultanée) selon les étapes suivantes :
- Dépôt d'une couche de métal 22 (Titane) d'épaisseur 500 nm
   Sous Argon à 1 10⁻² mbar/ pendant 5 min
- Photolithographie de résine photosensible positive (PFR7790 de chez JSR) ; épaisseur 1.1 µm
   Vitesse d'étalement : 4500 tr/min pendant 30 sec
   Recuit « doux » sur plaque chauffante (« Soft Bake ») : 3 minutes à 110°C
   Exposition à travers un masque pendant 4 secondes à 6,5 mW/cm² de puissance lumineuse UV
   Développement de la résine (développeur PRD238 de chez OMG) pendant 1min
   Rinçage à l'Eau Déionisée
   Recuit sur plaque chauffante (« Hard Bake ») : 3min à 110°C
- Gravure liquide (eau oxygénée à 30%) de la couche 22 de Titane
   Pendant 3 min / Rinçage à l'Eau Déionisée
- Gravure Plasma Oxygène de la couche de polymère 11 à travers la gravure de la couche de titane 22 pour former chaque trou 21
- Enlèvement de la couche de Titane 22 : utilisation, comme dissolvant, d'eau oxygénée à 30%

### Retrait du support rigide 10

Quelle que soit la variante du premier mode de réalisation de procédé selon l'invention (avec l'antenne 14 ou avec les trous 21), après le dépôt de la couche de polymère 11, on retire du support rigide 10 la superposition de couches de capteur, de sorte que les couches de capteur recouvertes par la couche de polymère 11 ne soient plus portées par le support rigide 10.

Pour retirer du support rigide 10 les couches de capteur, on détruit au moins partiellement le support rigide 10.

En référence aux figures 16 à 20, on procède de la manière suivante :
- Dépôt d'une couche métal 23 (aluminium) d'épaisseur 500 nm Sous Argon à 1 10⁻² mbar/ pendant 5 min
- Photolithographie de résine photosensible positive (PFR7790 de chez JSR) ; épaisseur 1.1 µm
   Vitesse d'étalement : 4500 tr/min pendant 30 secondes
   Recuit « doux » sur plaque chauffante (« Soft Bake ») : 3 minutes à 110°C
   Exposition à travers un masque pendant 4 secondes à 6,5 mW/cm² de puissance lumineuse UV
   Développement de la résine (développeur PRD238 de chez OMG) pendant 1min
   Rinçage à l'Eau Déionisée
   Recuit sur plaque chauffante (« Hard Bake ») : 3min à 110°C
- Gravure liquide (Al etch ANT 760-30-150 de chez OMG), Attaque de la couche d'aluminium 23 (une protection de la face avant est réalisée afin de pas graver l'aluminium en face avant, ex : dépôt de résine)
   Pendant 3 min / Rinçage à l'Eau Déionisée
- Enlèvement de la résine : utilisation, comme dissolvant, d'Acétone puis isopropanol alcool puis eau déionisée.
- Gravure DRIE (gravure profonde par ions réactifs ou « Deap Reactive Ion Etching ») SF6 (Hexafluorure de soufre) / C4F8 (Octafluorocyclobutane) de la couche de silicium 63, arrêt par la couche d'oxyde 13 (résultat obtenu illustré sur la figure 17)
- Gravure RIE (gravure par ions réactifs ou « Reactive Ion Etching ») SF6 (Hexafluorure de soufre) et CHF3 (Trifluorométhane) de la couche d'oxyde 13 (résultat obtenu illustré sur la figure 18)
- Dépôt sur la face arrière (i.e. ou se trouvait le support rigide 10 avant d'être partiellement détruit) d'une nouvelle couche 24 de polymère (de préférence même polymère que pour la couche 11) ou de colle pour créer une surface autocollante (résultat obtenu illustré sur la figure 19)
- découpe des structures : on retire ce qui reste des couches 23, 63 et 13 (résultat obtenu illustré sur la figure 20).

Toutes les figures 16 à 20 (ainsi que les suivantes 21 à 23) sont représentés avec des trous 21 hachurés et un contour d'antenne 14 en pointillés, selon que l'on considère que ces trous sont creusés (variante sans antenne 14) ou que ces trous sont remplis de polymère et n'ont pas été creusés (variante avec antenne 14).

Sur la figure 20, on remarque que la deuxième couche 24 de polymère est réalisée (après le retrait de l'au moins une partie du support 10) de sorte que_les couches de capteur soient encapsulées (c'est-à-dire contenues et de préférence enfermées) entre la première couche de polymère 11 et la deuxième couche de polymère 24.

### Autres variantes du premier mode de réalisation de procédé selon l'invention.

En référence aux figures 21 et 23, la couche de rigidification 12 peut être fabriquée avant la première électrode 6, 7 et la deuxième électrode 8, 9 (et donc avant la couche 19 isolante).

Lors de la fabrication du capteur 1, la couche de rigidification 12 n'est donc pas située entre les électrodes, 6, 7, 8, 9 et la couche de polymère 11 (cas précédent de la figure 20) mais est située entre les électrodes 6, 7, 8, 9 et le support rigide 10.

En référence aux figures 22 et 23, la couche 13 d'oxyde de silicium peut faire partie des couches de capteur ; dans ce cas, elle n'est pas détruite ou retirée.

### Premier procédé d'utilisation du capteur 1.

Un premier mode de réalisation de procédé d'utilisation du capteur 1 selon l'invention est un mode de réalisation « passif » ne nécessitant pas d'excitation externe au capteur 1 à part le signal devant être détecté.

Dans ce premier mode de réalisation :
- on applique (par exemple par une surface autocollante 24) le capteur 1 sur un objet (corps humain ou animal, ou objet inerte ni humain ni animal) puis
- pour chaque pulsation ou vibration ou déformation dudit objet, la pulsation ou vibration ou déformation déforme la couche piézoélectrique 5, engendrant une variation de charge électrique dans les électrodes 6, 7 ; 8, 9.
- pour chaque variation de charge électrique, on détecte cette variation de charge électrique.

Dans le cas particulier de pulsations ou vibrations ou déformations périodiques, on déduit ensuite, à partir de cette détection, une fréquence ou une période temporelle de ces pulsations ou vibrations ou déformations périodiques.

Par exemple:
- on applique (par exemple via une surface autocollante) le capteur 1 sur un corps humain ou animal (à proximité d'une veine ou artère), puis
- pour chaque pulsation cardiaque dudit corps parmi plusieurs pulsations, la pulsation cardiaque déforme la veine et la peau environnante ce qui entraine une déformation de la couche piézoélectrique 5, engendrant une variation de charge électrique dans les électrodes 6, 7 ; 8, 9 (proportionnelle à la déformation de la veine (amplitude et temps)).
- pour chaque variation de charge électrique, on détecte cette variation de charge électrique, et
- on déduit, à partir de ces détections, une fréquence ou une période cardiaque du corps.

### Deuxième procédé d'utilisation du capteur 1 (avec antenne 14 intégrée ou non).

Un deuxième mode de réalisation de procédé d'utilisation du capteur 1 selon l'invention est un mode de réalisation « actif » nécessitant une excitation externe au capteur 1 en plus du signal devant être détecté.

Dans ce deuxième mode de réalisation :
- on applique (par exemple par une surface autocollante 24) le capteur 1 sur objet (corps humain ou animal, ou objet inerte ni humain ni animal), puis
- on émet un champ électromagnétique 26 par une électronique d'excitation 25, puis
- le champ électromagnétique 26 est capté par l'antenne 14 ou par une antenne connectée aux électrodes 6, 7 et 8, 9 à travers les trous 21, engendrant dans cette antenne un courant électrique à une fréquence f1 égale à une fréquence f0 de résonance du capteur au repos (i.e. en l'absence de contrainte et de déformation de la couche piézoélectrique 5 qui est au repos) ou à une harmonique de cette fréquence f0 de résonnance,
- pour chaque pulsation ou vibration ou déformation dudit objet parmi plusieurs pulsations ou vibrations ou déformations, la pulsation ou vibration ou déformation déforme la couche piézoélectrique 5, provoquant une variation Df de la fréquence de résonance,
- pour chaque variation Df de la fréquence de résonance, on réceptionne par une électronique de mesure 25 un signal 27 oscillant à une fréquence f1 + Df,
- on déduit, à partir de ces détections, une fréquence ou une période temporelle des pulsations ou vibrations ou déformations.

Par exemple :
- on applique (par exemple via une surface autocollante) le capteur sur un corps humain ou animal (à proximité d'une veine ou artère), puis
- on émet un champ électromagnétique 26 par une électronique d'excitation 25, puis
- le champ électromagnétique 26 est capté par l'antenne 14 ou par une antenne connectée aux électrodes 6, 7 et 8, 9 à travers les trous 21, engendrant dans cette antenne un courant électrique à une fréquence f1 égale à une fréquence f0 de résonance du capteur au repos (i.e. en l'absence de contrainte et de déformation de la couche piézoélectrique 5 qui est au repos) ou à une harmonique de cette fréquence f0 de résonnance,
- pour chaque pulsation cardiaque dudit corps parmi plusieurs pulsations, la pulsation cardiaque déforme la veine et la peau environnante ce qui entraine une déformation de la couche piézoélectrique 5, provoquant une variation Df de la fréquence de résonance,
- pour chaque variation Df de la fréquence de résonance, on réceptionne par une électronique de mesure 25 un signal 27 oscillant à une fréquence f1 + Df,
- on déduit, à partir de ces détections, une fréquence ou une période cardiaque du corps.

L'électronique d'excitation et l'électronique de mesure est illustrée sur les figures 24 et 25.

En référence à la figure 24, le capteur 1 est excité à sa fréquence fondamentale de résonance (mécanique) ou à une harmonique (mécanique).

A ces fréquences le capteur 1 se comporte comme un filtre RLC série en parallèle à une capacité C₀ statique.

Un amplificateur à gain contrôlé 28 est rebouclé sur lui-même à travers le primaire d'un transformateur (mutuelle inductance entre les deux spires), le capteur étant branché au secondaire.

Le circuit se comporte comme un oscillateur dont la fréquence dépend de la fréquence de fonctionnement du capteur 1 (fréquence fondamentale de résonance ou harmonique mécanique).

La variation de fréquence est due à la variation de contrainte mécanique appliquée au capteur.

Une utilisation In-vivo de ce capteur peut être envisagée en choisissant judicieusement les matériaux utilisés et en encapsulant le capteur dans un packaging spécifique (exemple « Titanium polymer » en couche mince)

En référence à la figure 25, on a une transformation du signal sinusoïdal 27 en signal carré à l'aide d'un comparateur, puis une mesure de la fréquence f₀ + Df à l'aide d'un compteur, une détermination de la valeur de la fréquence sous format numérique, puis un traitement du signal.

La figure 26 présente les résultats d'une utilisation d'un capteur selon l'invention. Un capteur fabriqué selon le procédé de l'invention permet de mesurer la déformation artérielle induite par l'influx sanguin (onde de pouls) et ainsi apporte une information en accord avec l'électrocardiographie ou ECG (identification de l'activité électrique du coeur). Cette mesure reposant sur des amplitudes physiques, le capteur peut apporter des informations supplémentaires inaccessibles jusqu'à présent telles que les contraintes appliquées aux artères (élasticité, encombrement...). En outre, étant donné la faible épaisseur du capteur, notamment des couches de polymère l'encapsulant et sa souplesse, il permet une mesure sensible et en continu (e.g. utilisable lors des activités d'un sujet).

La partie A de la figure 26 est une courbe d'ECG obtenue sur un sujet sain de 30 ans par des électrodes commerciales de type COMEPA et un amplificateur d'instrumentation médicale du commerce (axe des abscisses : unité arbitraire de temps ; axe des ordonnées : unité arbitraire).

La partie B de la figure 26 est une courbe obtenue sur ce même sujet simultanément à la partie A avec un capteur selon l'invention correspondant au mode de réalisation illustré sur la figure 20 (mais sans antenne 14), en mettant en oeuvre un procédé de mesure venant d'être décrit, ce capteur étant collé par un sparadrap sur la veine jugulaire de ce sujet (axe des abscisses : unité arbitraire de temps ; axe des ordonnées : unité arbitraire).

Bien sûr, l'invention n'est pas limitée aux exemples qui viennent d'être décrits et de nombreux aménagements peuvent être apportés à ces exemples sans sortir du cadre de l'invention.

Par exemple, la forme de la couche piézoélectrique 5 peut avoir, vue de dessus, pas nécessairement une forme de poutre, mais peut avoir une forme de croix, une forme circulaire ou une forme de doigts interdigités.

De plus, on peut remplacer les couches de silicium 63 et d'oxyde de silicium 13 par un support rigide 10 en verre.

Il est en outre possible de ne pas retirer la totalité du support rigide 10 mais plutôt de laisser une partie du support rigide 10 (cette partie restante n'ayant alors plus pour rôle de porter les couches de capteur) par exemple en dessous de la couche piézoélectrique afin de laisser une masse pour d'autre mode de fonctionnement des capteurs (ex: microphone).

Dans une variante de la figure 19, la couche de polymère 24 recouvre également les parties numérotées 10, 63 et 23.

Dans une variante de chacune des figures 20, 21, 22 et 23, (après découpe) la « largeur » (définie horizontalement dans le plan de ces figures) de la couche de polymère 11 est réduite à la « largeur » de la couche de polymère 24 qui définit la taille du capteur.

Bien entendu, les différentes caractéristiques, formes, variantes et modes de réalisation de l'invention peuvent être associées les unes avec les autres selon diverses combinaisons dans la mesure où elles ne sont pas incompatibles ou exclusives les unes des autres. En particulier toutes les variantes et modes de réalisation décrits précédemment sont combinables entre eux.

Par exemple, un procédé de fabrication selon l'invention peut comprendre la « libération » des électrodes (réalisation des trous 21) et la fabrication de l'antenne 14, les trous 21 et l'antenne 14 n'étant pas incompatibles.

## Revendications

1. Procédé de fabrication d'un capteur piézoélectrique (1), comprenant les étapes suivantes :
- on fabrique, sur un support (10), une superposition de couches de capteur (2, 4, 5, 12, 19), les couches de capteur comprenant une couche de matière piézoélectrique (5) comprise entre une première électrode (6, 7) et une deuxième électrode (8, 9), la première électrode n'étant pas en contact de la deuxième électrode, puis
- alors que les couches de capteur (2, 4, 5, 12, 19) sont toujours portées par le support (10), on recouvre les couches de capteur par une première couche de polymère (11), puis
- on retire, de la superposition de couches de capteur, au moins une partie du support (10),
**caractérisé en ce que** la superposition de couches de capteur comprend une première couche conductrice (2) et une deuxième couche conductrice (4), chacune des première (2) et deuxième (4) couches conductrices comprenant deux parties, une première partie (6) de la première couche conductrice étant en contact avec une première partie (7) de la deuxième couche conductrice, une deuxième partie (8) de la première couche conductrice étant en contact avec une deuxième partie (9) de la deuxième couche conductrice, la première électrode (6, 7) étant formé par la première partie de la première couche conductrice et par la première partie de la deuxième couche conductrice, la deuxième électrode (8, 9) étant formé par la deuxième partie de la première couche conductrice et par la deuxième partie de la deuxième couche conductrice, la couche de matière piézoélectrique (5) étant comprise au moins en partie entre la première couche conductrice et la deuxième couche conductrice.

2. Procédé selon la revendication 1, **caractérisé en ce qu'**on fabrique, après le retrait de l'au moins une partie du support, une deuxième couche (24) de polymère de sorte que les couches de capteur (2, 4, 5, 12, 19) soient encapsulées entre la première couche de polymère (11) et la deuxième couche de polymère (24).

3. Procédé selon la revendication 1 ou 2, **caractérisé en ce qu'**on retire, de la superposition de couches de capteur, la totalité du support (10).

4. Procédé selon l'une quelconque des revendications précédentes, **caractérisé en ce que** le support (10) est un support rigide ayant, à des conditions standards ambiantes de température de 25 °C et de pression absolue de 1 bar (0.986 atm), un module d'Young supérieur à 100 GPa.

5. Procédé selon l'une quelconque des revendications précédentes, **caractérisé en ce qu'**il comprend en outre une fabrication d'une antenne reliant la première électrode à la deuxième électrode, ladite antenne faisant un ou plusieurs tours de manière à dessiner une boucle fermée.

6. Procédé selon la revendication 5, **caractérisé en ce que** la couche piézoélectrique (5) a une forme de poutre s'étendant longitudinalement et **en ce que** la poutre s'étend longitudinalement à partir du périmètre de la boucle et vers l'intérieur de la boucle.

7. Procédé selon l'une quelconque des revendications 5 à 6, **caractérisé en ce que** les couches de capteur comprennent une couche isolante, réalisée entre l'antenne et la première électrode et réalisée entre l'antenne et la deuxième électrode, au niveau de zones où l'antenne est superposée à la première électrode ou à la deuxième électrode.

8. Procédé selon la revendication 7, **caractérisé en ce que** la couche isolante est réalisée dans une matière isolante électriquement, ayant une résistivité électrique supérieure à 10³ ohm.cm à des conditions standards ambiantes de température de 25 °C et de pression absolue de 1 bar (0.986 atm).

9. Procédé selon l'une quelconque des revendications précédentes, **caractérisé en ce que** les couches de capteur comprennent en outre une couche de rigidification (12) superposée à la couche piézoélectrique.

10. Procédé selon la revendication 9 considérée comme dépendante de la revendication 7 ou 8, **caractérisé en ce que** la couche de rigidification et la couche isolante sont réalisées dans une même matière au cours d'une même étape de dépôt de couche.

11. Procédé selon l'une quelconque des revendications précédentes, **caractérisé en ce que** l'épaisseur totale des couches de capteur (2, 4, 5, 12, 19) est inférieure à 3 micromètres.

12. Capteur (1) obtenu par un procédé de fabrication selon l'une quelconque des revendications précédentes.

13. Procédé d'utilisation d'un capteur obtenu selon l'une quelconque des revendications 1 à 11, **caractérisé en ce qu'**il comprend les étapes suivantes :
- on applique le capteur (1) sur un corps humain ou animal, puis
- pour chaque pulsation cardiaque dudit corps parmi plusieurs pulsations, la pulsation cardiaque déforme la couche piézoélectrique (5), engendrant une variation de charge électrique dans les électrodes (6, 7 ; 8, 9).
- pour chaque variation de charge électrique, on détecte cette variation de charge électrique, et
- on déduit, à partir de ces détections, une fréquence cardiaque du corps.

14. Procédé d'utilisation d'un capteur obtenu selon l'une quelconque des revendications 1 à 11, **caractérisé en ce qu'**il comprend les étapes suivantes :
- on applique le capteur sur un corps humain ou animal, puis
- on émet un champ électromagnétique par une électronique d'excitation, puis
- le champ électromagnétique est capté par une antenne connectée électriquement à la première électrode (6, 7) et à la deuxième (8, 9) électrode du capteur (1), engendrant dans l'antenne un courant électrique à une fréquence f1 égale à une fréquence f0 de résonance du capteur au repos ou à une harmonique de cette fréquence f0 de résonnance,
- pour chaque pulsation cardiaque dudit corps parmi plusieurs pulsations, la pulsation cardiaque déforme la couche piézoélectrique (5), provoquant une variation Df de la fréquence de résonance,
- pour chaque variation Df de la fréquence de résonance, on réceptionne par une électronique de mesure un signal oscillant à une fréquence f1 + Df,
- on déduit, à partir de ces détections, une fréquence cardiaque du corps.

## Patentansprüche

1. Verfahren zum Herstellen eines piezoelektrischen Sensors (1), umfassend die folgenden Schritte:
- Herstellen einer Übereinanderlagerung von Sensorschichten (2, 4, 5, 12, 19) auf einem Träger (10), wobei die Sensorschichten eine Schicht aus piezoelektrischem Material (5) zwischen einer ersten Elektrode (6, 7) und einer zweiten Elektrode (8, 9) umfassen, wobei die erste Elektrode nicht mit der zweiten Elektrode in Kontakt steht, dann
- Bedecken der Sensorschichten mit einer ersten Polymerschicht (11), während die Sensorschichten (2, 4, 5, 12, 19) weiterhin vom Träger (10) getragen werden, dann
- Entfernen zumindest eines Teils des Trägers (10) von der Übereinanderlagerung von Sensorschichten,
**dadurch gekennzeichnet, dass**
die Übereinanderlagerung von Sensorschichten eine erste leitende Schicht (2) und eine zweite leitende Schicht (4) enthält, wobei jede der ersten (2) und zweiten (4) leitenden Schichten zwei Teile enthält, wobei ein erster Teil (6) der ersten leitenden Schicht mit einem ersten Teil (7) der zweiten leitenden Schicht in Kontakt steht und ein zweiter Teil (8) der ersten leitenden Schicht mit einem zweiten Teil (9) der zweiten leitenden Schicht in Kontakt steht, wobei die erste Elektrode (6, 7) aus dem ersten Teil der ersten leitenden Schicht und dem ersten Teil der zweiten leitenden Schicht gebildet ist, wobei die zweite Elektrode (8, 9) aus dem zweiten Teil der ersten leitenden Schicht und aus dem zweiten Teil der zweiten leitenden Schicht gebildet ist, wobei die Schicht aus piezoelektrischem Material (5) zumindest teilweise zwischen der ersten leitenden Schicht und der zweiten leitenden Schicht liegt.

2. Verfahren nach Anspruch 1, **dadurch gekennzeichnet, dass** nach dem Entfernen des zumindest einen Teils des Trägers eine zweite Polymerschicht (24) hergestellt wird, so dass die Sensorschichten (2, 4, 5, 12, 19) zwischen der ersten Polymerschicht (11) und der zweiten Polymerschicht (24) eingekapselt werden.

3. Verfahren nach Anspruch 1 oder 2, **dadurch gekennzeichnet, dass** der gesamte Träger (10) von der Übereinanderlagerung von Sensorschichten entfernt wird.

4. Verfahren nach einem der vorangehenden Ansprüche, **dadurch gekennzeichnet, dass** der Träger (10) ein starrer Träger mit einem Elastizitätsmodul von über 100 GPa bei Standardumgebungsbedingungen mit einer Temperatur von 25 °C und einem Absolutdruck von 1 bar (0,986 atm) ist.

5. Verfahren nach einem der vorangehenden Ansprüche, **dadurch gekennzeichnet, dass** es ferner ein Herstellen einer Antenne umfasst, die die erste Elektrode mit der zweiten Elektrode verbindet, wobei die Antenne eine oder mehrere Windungen macht, so dass eine geschlossene Schleife gebildet wird.

6. Verfahren nach Anspruch 5, **dadurch gekennzeichnet, dass** die piezoelektrische Schicht (5) eine sich in Längsrichtung erstreckende Balkenform hat und dass der Balken sich in Längsrichtung vom Umfang der Schleife bis zum Inneren der Schleife erstreckt.

7. Verfahren nach einem der Ansprüche 5 bis 6, **dadurch gekennzeichnet, dass** die Sensorschichten eine Isolierschicht enthalten, die zwischen der Antenne und der ersten Elektrode und zwischen der Antenne und der zweiten Elektrode an Bereichen ausgebildet ist, an denen die Antenne der ersten Elektrode oder der zweiten Elektrode überlagert ist.

8. Verfahren nach Anspruch 7, **dadurch gekennzeichnet, dass** die Isolierschicht aus einem elektrisch isolierenden Material mit einem spezifischen elektrischen Widerstand von über 10³ Ohm.cm bei Standardumgebungsbedingungen mit einer Temperatur von 25 °C und einem Absolutdruck von 1 bar (0,986 atm) ausgebildet wird.

9. Verfahren nach einem der vorangehenden Ansprüche, **dadurch gekennzeichnet, dass** die Sensorschichten ferner eine Versteifungsschicht (12) enthalten, die der piezoelektrischen Schicht überlagert ist.

10. Verfahren nach Anspruch 9 in Abhängigkeit von Anspruch 7 oder 8, **dadurch gekennzeichnet, dass** die Versteifungsschicht und die Isolierschicht aus einem gleichen Material im Laufe desselben Schritts der Schichtabscheidung ausgebildet werden.

11. Verfahren nach einem der vorangehenden Ansprüche, **dadurch gekennzeichnet, dass** die Gesamtdicke der Sensorschichten (2, 4, 5, 12, 19) geringer als 3 Mikrometer ist.

12. Sensor (1), erhalten durch ein Herstellungsverfahren nach einem der vorangehenden Ansprüche.

13. Verfahren zum Verwenden eines Sensors, erhalten nach einem der Ansprüche 1 bis 11, **dadurch gekennzeichnet, dass** es die nachfolgenden Schritte umfasst:
- der Sensor (1) wird an den Körper eines Menschen oder Tieres angelegt,
- bei jedem Herzschlag des Körpers unter mehreren Herzschlägen wird die piezoelektrische Schicht (5) durch den Herzschlag verformt, was zu einer Änderung der elektrischen Ladung in den Elektroden (6, 7; 8, 9) führt,
- bei jeder Änderung der elektrischen Ladung wird diese Änderung der elektrischen Ladung erfasst, und
- ausgehend von diesen Erfassungen wird eine Herzfrequenz des Körpers abgeleitet.

14. Verfahren zum Verwenden eines Sensors, erhalten nach einem der Ansprüche 1 bis 11, **dadurch gekennzeichnet, dass** es die nachfolgenden Schritte umfasst:
- der Sensor wird an den Körper eines Menschen oder Tieres angelegt,
- ein elektromagnetisches Feld wird über eine Anregungselektronik erstellt,
- das elektromagnetische Feld wird von einer Antenne erfasst, die elektrisch mit der ersten Elektrode (6, 7) und der zweiten Elektrode (8, 9) des Sensors (1) verbunden ist, wodurch in der Antenne ein elektrischer Strom mit einer Frequenz f1 erzeugt wird, die gleich einer Resonanzfrequenz f0 des ruhenden Sensors oder einer Harmonischen dieser Resonanzfrequenz f0 ist,
- bei jedem Herzschlag des Körpers unter mehreren Herzschlägen wird die piezoelektrische Schicht (5) durch den Herzschlag verformt, was zu einer Änderung Df der Resonanzfrequenz führt,
- bei jeder Änderung Df der Resonanzfrequenz wird ein mit einer Frequenz f1 + Df oszillierendes Signal von einer Messelektronik empfangen,
- ausgehend von diesen Erfassungen wird eine Herzfrequenz des Körpers abgeleitet.

## Claims

1. Process for producing a piezoelectric sensor (1), comprising the following steps:
- producing, on a support (10), a stack of sensor layers (2, 4, 5, 12, 19), the sensor layers comprising a layer of piezoelectric material (5) comprised between a first electrode (6, 7) and a second electrode (8, 9), the first electrode not being in contact with the second electrode, then
- while the sensor layers (2, 4, 5, 12, 19) are still carried by the support (10), coating the sensor layers with a first layer (11) of polymer, then
- removing at least a part of the support (10) from the stack of sensor layers
**characterized in that** the stack of sensor layers comprises a first conductive layer (2), and a second conductive layer (4), each of the first (2) and second (4) conductive layers comprising two parts, a first part (6) of the first conductive layer being in contact with a first part (7) of the second conductive layer, a second part (8) of the first conductive layer being in contact with a second part (9) of the second conductive layer, the first electrode (6, 7) being formed by the first part of the first conductive layer and by the first part of the second conductive layer, the second electrode (8, 9) being formed by the second part of the first conductive layer and by the second part of the second conductive layer, the layer of piezoelectric material (5) being comprised at least in part between the first conductive layer and the second conductive layer.

2. Process according to claim 1, **characterized in that**, after the removal of the at least a part of the support, a second layer (24) of polymer is produced, so that the sensor layers (2, 4, 5, 12, 19) are encapsulated between the first polymer layer (11) and the second polymer layer (24).

3. Process according to claim 1 or 2, **characterized in that** all of the support (10) is removed from the stack of sensor layers.

4. Process according to any one of the preceding claims, **characterized in that** the support (10) is a rigid support having, under standard ambient conditions of a temperature of 25°C and absolute pressure of 1 bar (0.986 atm.), a Young's modulus greater than 100 GPa.

5. Process according to any one of the preceding claims, **characterized in that** it also comprises the production of an antenna linking the first electrode to the second electrode, said antenna making one or more turns so as to create a closed loop.

6. Process according to claim 5, **characterized in that** the piezoelectric layer (5) is in the form of a bar extending longitudinally and **in that** the bar extends longitudinally from the perimeter of the loop and towards the inside of the loop.

7. Process according to any one of claims 5 to 6, **characterized in that** the sensor layers comprise an insulating layer, produced between the antenna and the first electrode and produced between the antenna and the second electrode, at zones where the antenna is superimposed on the first electrode or the second electrode.

8. Process according to claim 7, **characterized in that** the insulating layer is produced from an electrically insulating material, having an electrical resistivity greater than 10³ ohm.cm under standard ambient conditions of a temperature of 25°C and absolute pressure of 1 bar (0.986 atm).

9. Process according to any one of the preceding claims, **characterized in that** the sensor layers also comprise a stiffening layer (12) superimposed on the piezoelectric layer.

10. Process according to claim 9 considered as dependent on claim 7 or 8, **characterized in that** the stiffening layer and the insulating layer are produced from one and the same material during one and the same layer deposition step.

11. Process according to any one of the preceding claims, **characterized in that** the total thickness of the sensor layers (2, 4, 5, 12, 19) is less than 3 micrometres.

12. Sensor (1) obtained by a production process according to any one of the preceding claims.

13. Process for using a sensor obtained according to any one of claims 1 to 11, **characterized in that** it comprises the following steps:
- the sensor (1) is applied on a human or animal body, then
- for each heartbeat of said body among several beats, the heartbeat deforms the piezoelectric layer (5), generating a variation in electric charge in the electrodes (6, 7; 8, 9).
- for each variation in electric charge, this variation in electric charge is detected, and
- a heart rate of the body is deduced from these detections.

14. Process for using a sensor obtained according to any one of the claims 1 to 11, **characterized in that** it comprises the following steps:
- the sensor is applied on a human or animal body, then
- an electromagnetic field is emitted by excitation electronics, then
- the electromagnetic field is picked up by an antenna electrically connected to the first electrode (6, 7) and to the second (8, 9) electrode of the sensor (1), generating an electric current in the antenna, at a frequency f1 equal to a resonance frequency f0 of the inactive sensor or to a harmonic of this resonance frequency f0,
- for each heartbeat of said body among several beats, the heartbeat deforms the piezoelectric layer (5), causing a variation Df in the resonance frequency,
- for each variation Df in the resonance frequency, a signal oscillating at a frequency f1 + Df is received by measurement electronics,
- a heart rate of the body is deduced from these detections.
